# EUROPEAN PATENT SPECIFICATION

(11) **EP 2 222 347 B1**
(45) Date of publication and mention of the grant of the patent: **31.07.2013**
(21) Application number: 08853269.2
(22) Date of filing: 26.11.2008
(51) Int. Cl.: A61L 26/00, A61L 27/36, A61L 27/52

(54) **BIOACTIVE AND RESORBABLE SOYBEAN-BASED BIOMATERIALS**
BIOAKTIVE UND RESORBIERBARE BIOMATERIALIEN AUF BASIS VON SOJABOHNEN
BIOMATÉRIAUX RÉSORBABLES ET BIOACTIFS À BASE DE SOJA

(30) Priority: 26.11.2007 GB 0723102
(43) Date of publication of application: 01.09.2010
(73) Proprietor: University of Brighton, Brighton, Sussex BN2 4AT (GB)
(72) Inventor: AMBROSIO, Luigi, I-80125 Naples (IT); NICOLAIS, Luigi, I-80125 Naples (IT); SALVAGE, Jonathan Peter, Brighton, East Sussex BN2 4GJ (GB); SANTIN, Matteo, Brighton, East Sussex BN2 4GJ (GB); STANDEN, Guy, Brighton, East Sussex BN2 4GJ (GB)
(74) Representative: Gaunt, Robert John
(86) International application number: PCT/GB2008/051117
(87) International publication number: WO 2009/068913

(56) References cited:
- WO-A1-02/10261
- WO-A1-95/10512
- US-A1- 2005 123 662
- US-A1- 2007 054 031
- US-A1- 2007 077 305

## Description

This invention relates to a method for producing soybean-based biomaterials. The resulting biomaterials have a range of biomedical uses and are particularly desirable because of their isoflavone content.

As understood by persons skilled in the art, a biomaterial is a non-viable material used in a medical device (such as an implant) intended to interact with biological systems. The future of regenerative medicine closely depends on the availability of novel biomaterials able to (i) intervene in the tissue regeneration processes and enhance the formation of new tissue showing physiological morphology and (ii) degrade in time leaving space to the newly-formed tissue. None of the available biomaterials is able to fulfil these objectives unless very expensive and unstable growth factors are loaded into the material bulk and their controlled delivery optimised, or unless stem and differentiated cells are encapsulated (1, 2).

The regeneration of soft tissues (e.g. blood vessels and skin) as well as of hard tissues (i.e. bone and cartilage) has been pursued so far by the use of synthetic or natural polymers and ceramics able to provide a scaffold for the in-growth of new tissue at the site of injury (2, 3 and 4). However, the currently available biomaterials are not able to act selectively on the main phases of the tissue regeneration process which are: (i) the coagulation cascade, (ii) the inflammatory response, (iii) the tissue cell differentiation and (iv) the synthesis of new extra-cellular matrix.

In addition, the presence of the implant, recognised as a foreign body by the host tissue, normally triggers an inflammatory response (5). As a consequence of this foreign body response, (i) permanent implants are not always completely integrated with the growing tissue, but they are encapsulated by a fibrous capsule; and (ii) the biodegradation/bioresorption rate of temporary implants may be affected and not in tune with the growth rate of the new tissue. The latter is the case with the biodegradable biomaterials accepted by the Food and Drug Administration, such as the various formulations of poly(lactic/glycolic) acids (PGLA) (6). In the case of bone applications, for example, the relatively slow degradation of PGLA delays tissue regeneration (7). Even when the degradation time is reduced as in the case of some PGLA formulations, the morphology of the bone formed around the implant shows non-physiological features (i.e. cortical bone in place of trabecular bone) suggesting an altered mechanism of tissue regeneration (7). The non-physiological healing observed after implantation of these materials has also been ascribed to the inflammatory response elicited by the material surface physico-chemical properties and, in the case of PGLA, to the degradation products (8).

Ceramics such as hydroxyapatite (HA), calcium phosphate (CaP) cements and bioglasses have also been developed mainly for bone regeneration applications. Although not biodegradable, these materials have shown a high osteointegrative potential (9, 10). In the case of HA and CaP cements, the osteointegrative potential seems to be generated by the material cell substrate properties which allow the colonisation of the surface by the bone cells, the osteoblasts (11).

In the case of bioglasses, a degree of bioactivity has been ascribed either to the release of elements (e.g. silicon) from the degrading material or by the entrapment and concentration of growth factors in the gel milieu formed at the interface during the bioglass degradation (12).

The performances of the ceramic materials are, however, limited only to certain applications because of their brittle and not malleable nature. Monolithic ceramics are difficult to handle during the implantation procedure, while ceramic coatings delaminate under the mechanical loadings (9, 13).

Natural polymers of protein or polysaccharide composition such as collagen, agarose, alginate, chitosan, fibrin glue, silk fibroin, hyaluronic acid and carboxymethylcellulose are also used as biodegradable biomaterials and some of them have been shown to have haemostatic properties and to support cell adhesion (14, 15 and 16). Fibrin glue, hyaluronic acid and collagen are natural components of a regenerating tissue and their performances in biomedicine have been demonstrated (14). However, concerns about their use as biomaterials are raised by their antigenic potential, by the risks of transmittable diseases and by the relatively high manufacturing costs. In the specific case of bone fillers, risks of transmittable diseases are also linked to the use of bone allografts, while autografts lead to a second operation on the patient at different sites to harvest bone and with limitations in the amounts available.

The use of soy-based biomaterials has also been suggested (17, 18). The research has been oriented towards the manufacture of soy protein hydrogels, films, membranes and fibres from the soybean protein fractions (18). Although very malleable, this type of biomaterial suffers from the same limitations as the other natural, biodegradable polymers; mainly an antigenic potential and an uncontrolled (not tuneable) rate of degradation (19). Furthermore, they do not include the isoflavone fraction which has a proven bioactivity on both immunocompetent cells and tissue cells such as osteoblasts and osteoclasts (20, 21). A patent has been filed that covers the use of genistein, one of the soy isoflavones, as a pharmaceutical agent to reduce bone loss in osteoporosis by inhibiting osteoclast acid activity (21).

For these reasons, more recently, a new class of soy-based biomaterials has been developed using de-fatted soybean curd which includes all the components of the soy: proteins, carbohydrates, minerals and isoflavones (22). This class of biomaterial can be formulated as films, membranes and granules either as a monolithic material or in combination with other conventional biomaterials. Moreover, this new class of soy-based biomaterials and their degradation products have shown many properties suitable for biomedical applications among which are to: (i) control the inflammatory response, (ii) have a controlled (tuneable) rate of degradation, (iii) favour cell activity and (iv) induce the deposition of a calcium phosphate mineral phase (22).

Although the soy protein-based biomaterials have been engineered in the form of hydrogels, films and fibres, they lack the recognised bioactivity of the soy isoflavones on tissue cells. Conversely, the soy-based biomaterials obtained from the de-fatted soybean curd-derived biomaterials have not been thus far formulated in the form of soft hydrogels suitable, for example, for implantation by injection. In addition, their method of manufacture depends on the preliminary preparation of a curd and it relies on the natural soybean isoflavone content without the possibility of modifying its levels.

Methods have been published which show the extraction of soybean protein concentrates rich in isoflavones (23). These methods have been optimised only for food industry purposes and have not been focused to the preparation of bioactive hydrogels for biomedical applications. Furthermore, no study has been performed on the co-extraction of the protein and carbohydrate fractions together with a tuned isoflavone content of the soy flour and curd from which the biomaterials are produced.

WO02/10261 from the same applicant discloses a compact or porous thermoplastic biomaterial produced from soybean de-fatted tofu which has been functionalized with one or more bioactive peptides. The biomaterial has been also subjected to thermosetting.

US2007/077305 discloses bioactive agents as peptides or proteins for delivery in gastrointestinal tract comprising soybean proteins which could serve as a competitive substrate and reduce the attack of proteases on the bioactive peptides or proteins.

US2007/054031 discloses a method of producing a soybean-based product comprising defatting soy flour, performing a solvent extraction to produce a biomaterial comprising variable levels of soy proteins, carbohydrates and isoflavones.

US2005/123662 discloses a process for producing a soybean saponin-containing material which comprises preparing a content of malonyl isoflavone glycoside being 25% by weight or more based on the total amount of isoflavones in said extract and an extraction ratio of soybean saponins from said starting soybeans being 60% by weight or more.

WO95/10512 refers to Soybean flakes from which the oil has been removed by solvent extraction. The flakes are extracted with an aqueous extractant having a pH of about 6.0 to about 10.0.

According to the present invention there is provided a method of producing a soybean-based biomaterial which is suitable for use in a biomedical product as mentioned in the claims.

According to a further aspect the present invention relates to the soybean-based biomaterial produced by the aforementioned method. For example, the biomaterial may be in the form of films, membranes, granules, hydrogels and pastes.

According to another aspect the present invention relates to the use of the soybean-based biomaterial produced by the aforementioned method in a biomedical product, and to such products themselves. As understood by persons skilled in the art, biomedical products are medical products or devices intended for use in tissue repair treatments, surgery and tissue regeneration. They do not include food products.

In contrast to the previously known techniques discussed above, the method of the present invention enables soybean-based biomaterials to be prepared directly from de-fatted soybean flour. This method is efficient and, most importantly, results in the production of biomaterials having a predetermined or controllable (tuned or tuneable) composition, e.g. the amount of isoflavones and/or other components contained in the biomaterials can be predetermined or controlled so as to be most suitable for the particular product's intended area of use.

The method of this invention involves extracting the soybean-based biomaterial from soy flour. It enables the levels of the soy proteins, carbohydrates and isoflavones present in the soy flour to be preserved (or varied as desired). This is in contrast to the previous approaches which focus on retaining soy proteins, but lose carbohydrates and isoflavones. By providing a method which enables the levels and proportions of all three of these components to be varied or modulated, the present invention makes it possible to control the physical and chemical characteristics and also the bioactivity of the biomaterial and, in consequence, the properties of the resulting biomedical products.

The resulting biomaterials can be used as monolithic materials or in combination among themselves or with other traditional polymeric, ceramic and metal materials in the form of blends, interpenetrating polymer networks and coatings. The biomaterials are bioactive as they:
(i) participate in the formation of the blood clot on the basis of the known ability of the soy protein to act as a substrate for transglutaminase enzymes (24, 25),
(ii) induce the synthesis of collagen in fibroblasts and osteoblasts,
(iii) induce osteoblast calcification and
(iv) inhibit the activity of osteoclasts.

Their range of applications includes, for example, as tissue defect fillers; wound dressings; scaffolds and guides for bone, skin and nerve regeneration; temporary barriers for use in dental or surgical procedures or to prevent post-surgical tissue adherence; anti-inflammatory agents; carriers for the delivery of drugs, bioactive peptides or plasmids; bioactive coatings for polymeric and metallic orthopaedic, neurological, gynaecological, urological, cranio-facial, dental and cardiovascular implants and as bioglue. The biomaterial can also be prepared in different formulations or dissolved in aqueous solutions to become soothers suitable for the treatment of irritated gums and skin. All these formulations can be used in clinical and veterinary applications.

The solvent extraction step in the method of this invention may be performed using, for example, a solvent system comprising methanol, ethanol, acetonitrile, acetone and water, or a mixture thereof. It is typically performed for a period of between 2 hours and 4 hours and at a temperature of between 30°C and 50°C. The method may, though need not necessarily, include one or more of the following additional steps:-
i) introducing a cross-linking agent;
ii) dialysis, for the purpose of controlling the level of carbohydrates in the biomaterial;
iii) thermosetting;
iv) blending, interpenetrating, grafting or otherwise combining the soybean-based biomaterial with one or more natural or synthetic biocompatible biomaterials to form a composite biodegradable or biostable material.

It will be understood that step iv) is an important option when the biomaterial is in the form of a hydrogel. In one embodiment, the hydrogel is in an injectable form.

The present invention will now be further illustrated by the following Examples (in which Example 1 describes the de-fatting process used in the subsequent Examples) and with reference to the accompanying figures, as follows:-
**Figure 1****.** FT-IR of the soy-based biomaterials: (a) soybean flour, (b) de-fatted soybean flour, (c) de-fatted and thermoset soybean biomaterial.
**Figure 2****.** FTI-IR of the freeze-dried extracted soy gel.
**Figure 3****.** HPLC-determined isoflavone levels of a typical soy-based gel: (a) glycosylated isoflavones, (b) non-glycosylated isoflavones.
**Figure 4****.** Typical soy-based hydrogels: (a) non-crosslinked gel, (b) genipin-crosslinked gel forming a bioglue.
**Figure 5****.** The rheological properties of the soybean-based hydrogels at a crosslinking agent concentration of 0.1 M: (a) Storage Modulus G' (b) Viscous Modulus G".
**Figure 6****.** Viscosity measurements for the soybean-based hydrogels at a crosslinking agent concentration of 0.1 M.
**Figure 7****.** Soy-based hydrogel yields obtained from different extraction conditions.
**Figure 8****.** HPLC-determined isoflavone levels of the soy-based hydrogels obtained from different extraction conditions: (a) daidzin, (b) daidzein, (c) genistin, (d) genistein.
**Figure 9****.** SEM of blends of soy-based hydrogels with other conventional biomaterials: (a) soy gel/soy granules/xanthan gum visual inspection, (b) soy gel/soy granule/xanthan gum SEM analysis, (c) soy gel/ nanocrystal hydroxyapatite, (d) soy gel/hydroxyapatite beads.
**Figure 10****.** Typical HPLC-determined isoflavone release from soy-based biomaterials. Legends show the xanthan gum/soy granules/soy hydrogel ratio in the different blends tested.
**Figure 11****.** A typical soy-gel formulation (injectable) for use as wound dressing or bone filler.
**Figure 12****.** Typical soy-based biomaterials induction of cell differentiation: (a) collagen staining of control fibroblasts after 1 day of culture, (b) collagen staining of soy-based biomaterials treated fibroblasts after 1 day of culture, (c) calcium staining of control osteoblasts after 2 days of culture, (d) calcium staining of soy-based biomaterials treated osteoblasts after 2 days of culture.
**Figure 13****.** Integration of soy-based biomaterials in the blood clot: (a) visual inspection of 2 different formulations, (b) light microscopy.
**Figure 14****.** Inhibitory effect of the soy-based biomaterials on osteoclast tartrate-resistant acid phosphatase: (a) control osteoclast, (b) soy-based biomaterial-treated osteoclast. Arrows indicate osteoclasts.
**Figure 15****.** Backscattering SEM of typical soy-based biomaterial coating around a biomedical device (cardiovascular stent): (a) coated cardiovascular stent, (b) soy gel-coated cardiovascular stent surface morphology, (c) uncoated cardiovascular stent surface morphology.

### Example 1 - Methods of biomaterial preparation from de-fatted soybean flour

### Methods

Soy flour was freeze-dried to remove water content. Soy flour was de-fatted following a method usually employed in the food industry (26, 27). Briefly, freeze-dried flour was suspended in hexane (1:5 ratio) at 30°C for 4 hours, in a shaking incubator at a 45 degree angle and 200 rpm to ensure effective solvent/flour mixing. The suspension was removed from the incubator and allowed to cool and settle for 10 - 15 minutes. When the flour had settled, the hexane fraction was discarded, fresh hexane added, and the flour re-suspended and allowed to settle for 10 - 15 minutes. The hexane washing was repeated three times to remove any lipid trace and the de-fatted flour was allowed to dry for 48 hours at room temperature. Lipid extraction was evaluated gravitometrically, whereas isoflavone content was assessed by HPLC. HPLC was performed using a Phenomenex Luna C₁₈ (2) - 150mm x 4.6mm (3µm particle size) column equipped with SecurityGuard Phenomenex (3µm) guard cartridge. The heater was set at 25°C. Chromatography was performed in a mobile phase consisting of a binary gradient (Solvent A: deionised water and 0.1 % acetic acid, Solvent B: acetonitrile and 0.1% acetic acid) which was pumped by Perkin Elmer Series 200 Ic binary gradient: a pump programmed to deliver the Sovent A/Solvent B mixtures at the following conditions 10/90 (0 mins) - 15/85 (0.1 mins) - 20/80 (4 mins) - 40/60 (9 mins) - 60/40 (0.1 mins) hold at 60/40 (4 mins). Total run time - 17.2 minutes. The eluted isoflavones were detected by a Shimadzu SPD-6A UV detector at 262nm, 2.56 AUFS, fast response. Chromatograms were obtained by a Shimadzu C-R5A Chromatopac integrator/chart recorder programmed with a: 150 µV/min slope, µV 10 /min drift, 50 µV min. peak area, 0 attenuation, at a speed of 4mm/min, and including area and baseline/integration print (no RT on chart). Samples were injected by autosampling using a Waters 717+ with 96 shell vial carousel autosampler with 250 µl inserts. The carousel was programmed with a 10 µl injection volume, 25°C injection temperature, 21 minute run and 3.5 minute report. To ensure baseline stability the total run time was 24.5 minutes.

The soy-based biomaterial was obtained without the preliminary curd preparation by thermosetting the de-fatted flour at 60°C, overnight. Through this method, biomaterials in the form of granules, films, membranes and blocks of different shapes, sizes and porosity can be obtained.

### Results

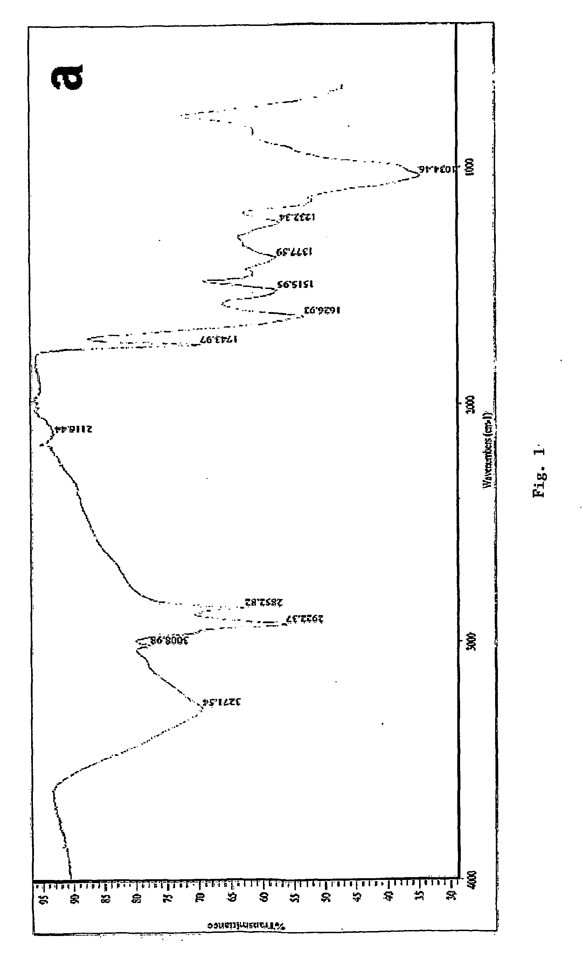

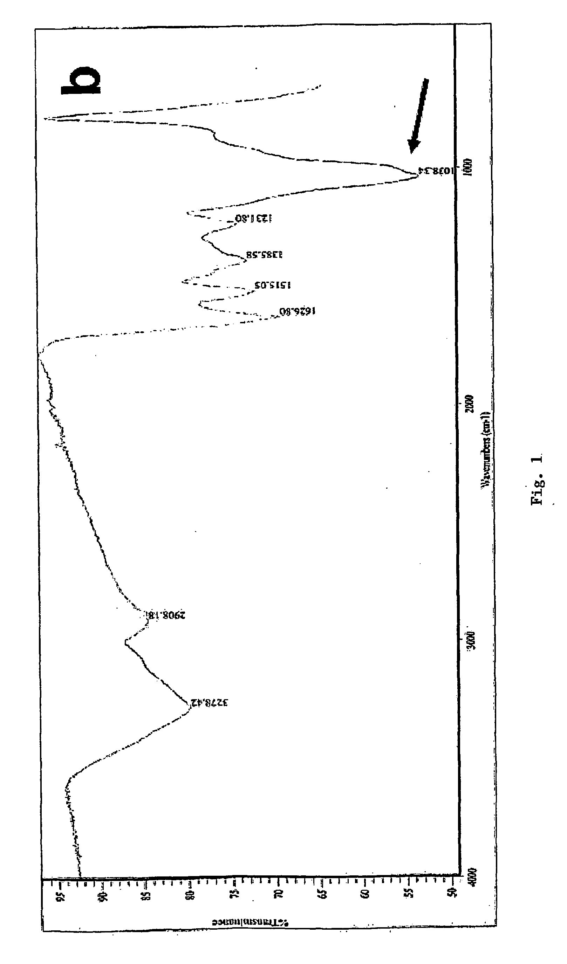

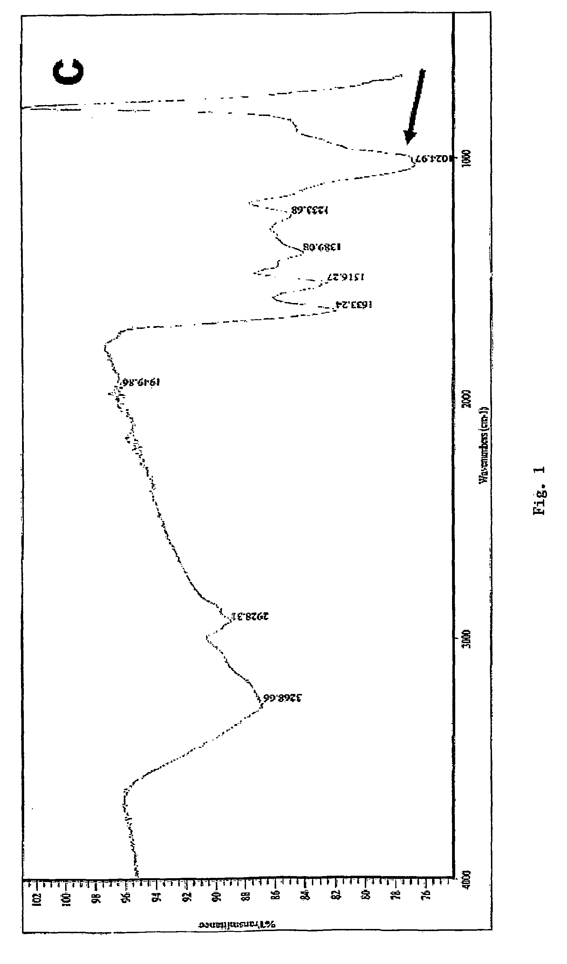

The conventional extraction method employed ensured a removal of soybean lipids of 0.175 g per gram of soy which corresponded to 18% lipid component of soy as reported in the literature. The content of the main soybean isoflavones after extraction showed only a minimal removal of genistin (0.7 mg/g soy flour) and no detectable co-extraction of the other isoflavones (e.g. genistein and daidzein). The effective removal of lipids was confirmed by FTIR which showed the lack of the lipids peaks at 2922, 2852 and 1734 cm⁻¹ (Figure 1 a and b). The efficacy of the thermal cross-linking adopted to transform the de-fatted soy flour into plastic is also proven by the shift of some of the soy protein peaks as well as by the change of their relative ratio (Figure 1 c). In particular, the amine I peak changed from 1626 to 1633 cm⁻¹, while the amine II peak (from 1515 to 1516 cm⁻¹) and the amine III peaks (from 1385 to 1389 cm⁻¹ and from 1231 to 1233 cm⁻¹) did not show any significant shift (Figures 1 b and c). However, the amine II peak showed a higher intensity and its ratio with the amine I peak changed. The peak at 1038 cm⁻¹, attributed to the carbohydrate fraction of the soy, was also shifted after thermosetting and the ratio with its shoulder changed as the shoulder intensity increased (Figures 1 b and c, arrows).

### Example 2 - Methods of preparation of soy-based hydrogel biomaterials from de-fatted soy flour

### Methods

The soy flour was de-fatted as described in Example 1. To prepare soybean-based hydrogels, the de-fatted flour was suspended in appropriate solvent systems (1:10 flour/solvent ratio). The solvent systems used included, but were not limited to: methanol, ethanol, acetonitrile, acetone, de-ionised water, 0.05N HCl or a mixture of the above at different ratios (e.g. ethanol/water 80:20). The sample was placed at a 45 degree angle in a shaking incubator (200 rpm) for different times (e.g. from 2 to 4 hours), at different temperatures (e.g. from 30°C to 50°C). The samples were cooled at room temperature and allowed to settle for 30 min. Supernatant was collected and centrifuged for 10 min at 2500 rpm, room temperature. The obtained supernatant was filtered through a clean glass syringe packed with glass wool. Solvent was evaporated under nitrogen flow followed by freeze-drying. Gels of different density were obtained by re-suspending the freeze-dried powder in de-ionised water or cross-linking agent solutions (e.g. 0.1 M CaCl₂ or MgCl₂ aqueous solution, dialdehyde-based aqueous solutions). Alternatively, the obtained gel, with or without cross-linking agent, underwent a further stabilisation step by thermosetting at 60°C, overnight.

In an alternative method, soybean-based hydrogels were obtained by simultaneous (contemporary) extraction during the soybean flour de-fatting process. The flour was agitated for 2 hours at 50°C in a co-solvent system such as, but not limited to, ethanol:water:hexane (80:20:10 ratio). The suspension was left to settle and cool for five minutes. The top hexane layer was discarded and the remaining supernatant and solids were separated by decantation. The solid was washed a few more times with a fresh solvent system as described above and the supernatants from the different extractions pooled. The filtered and pooled supernatants were evaporated under nitrogen flow followed by freeze-drying. The hexane supernatant was characterised for its lipid and isoflavone contents as described in Example 1.

Soybean-based hydrogels were obtained as described above in the presence and absence of cross-linking agent solutions and with or without thermosetting. The obtained powder and relative hydrogels with or without cross-linking were characterized for their overall composition by FTIR, for their protein content by the Bradford method, for their carbohydrate content by the Anthron method and for their isoflavone content by HPLC as described in Example 1.

The protein content of both raw materials and extract was determined by a conventional Bradford method. Briefly, soybean samples were dissolved in 0.1 N NaOH and incubated under shaking for 1hour, room temperature. After incubation, 100 µl of samples were mixed with 100 µl of the Bio-Rad protein reagent dye (Biorad, UK, catalogue n. 500-0006) in a 96-well plate and incubated for 5 min at room temperature. The absorbance of the samples at 595 nm was measured and the values transformed into protein concentration by a standard curve (R²= 0.995) obtained from bovine serum albumin (Sigma Aldrich, catalogue n. A7030) solutions in the range of 0 to 0.1 mg/ml. Experiments were performed in triplicate on samples from different batch preparations.

The Anthron method assay was performed to assess the total saccharide amount in both the starting raw material and the final soy extract obtained by the 80/20 ethanol/water solvent system. Briefly, samples were incubated with a freshly prepared Anthron solution (0.4 g, Acros Organics, UK) in 75% sulphuric acid (200 ml, Fisher Scientific, UK) at 130 °C for 10 min. The solution was chilled in ice and the total amount of carbohydrates in the samples was measured by spectrophotometry at 578 nm. A standard curve with a R² value of 0.998 was obtained by the absorbance reading of glucose standards in the range of 0 to 0.1 mg/ml. Experiments were performed in triplicate on samples from different batch preparations.

Viscosity as a function of shear rate, elastic and viscous moduli G' and G" were determined at 37°C, using a stress controlled rheometer (Bohilin Mod.Gemini) with a cone-plate tool. During the experiments, gels were kept in a contolled environment by a humidity chamber. The soybean-based hydrogels were tested at different cross-linking agent concentrations (0.1 M and 1.0 M CaCl₂) and by preparing the hydrogels with different water contents (100 mg of soybean extract powder reconstituted as hydrogel in either 50 or 80 µl of crosslinking solution).

### Results

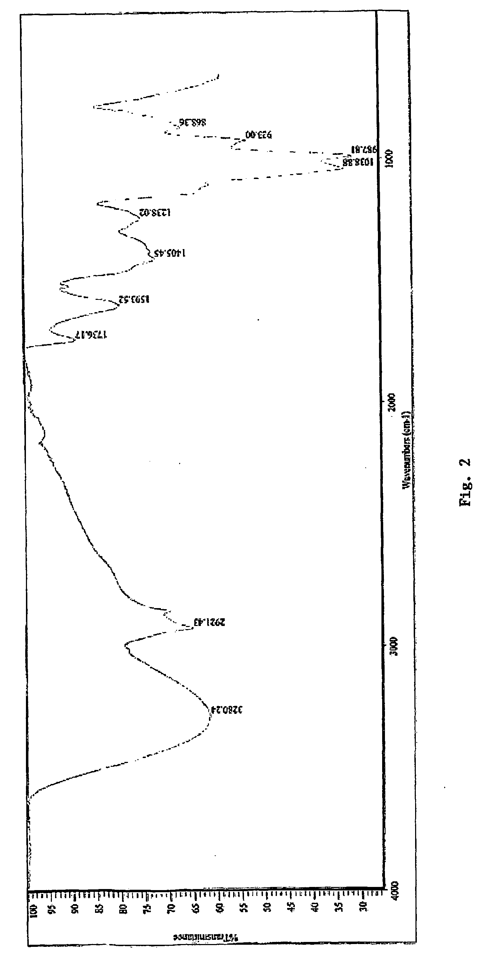

The hydrogel extraction performed simultaneously (contemporarily) to the de-fatting process showed de-fatting levels comparable to those obtained by the method described in Example 1. Figure 2 shows the FTIR of the soybean-based powder obtained after extraction, solvent evaporation and freeze-drying. Although conformational changes in the protein fraction could be observed, the overall protein and carbohydrate composition of the de-fatted soy flour was preserved (Figures 1 and 2).

The amount of both the protein and carbohydrate fractions in the extracts was quantified showing that the extraction procedure led to protein and carbohydrate concentrates (protein fraction = 56%, carbohydrate fraction = 35% by weight of dry powder). The extraction obtained contemporarily to the de-fatting process showed de-fatting levels comparable to those obtained by the sequential method.

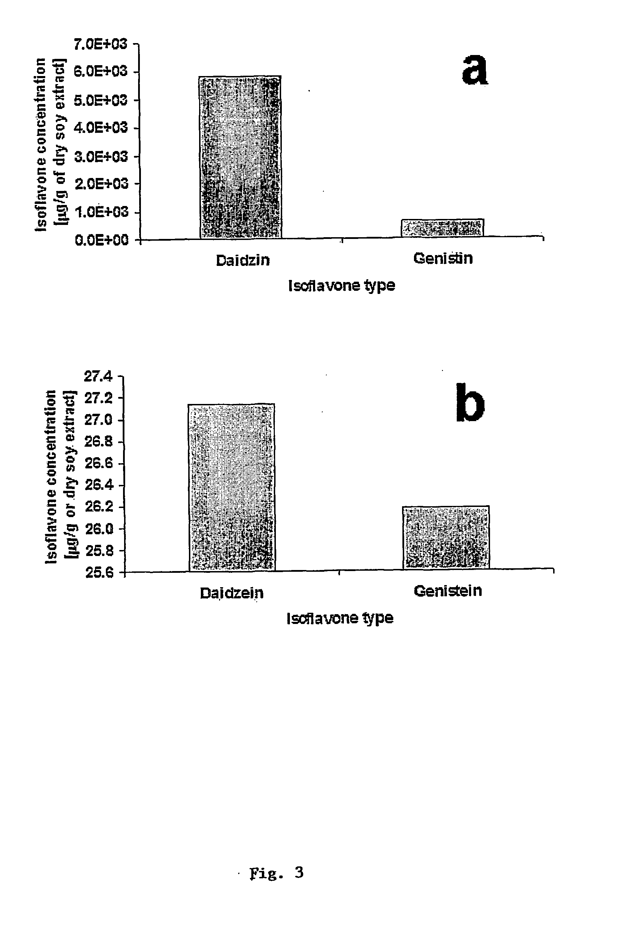

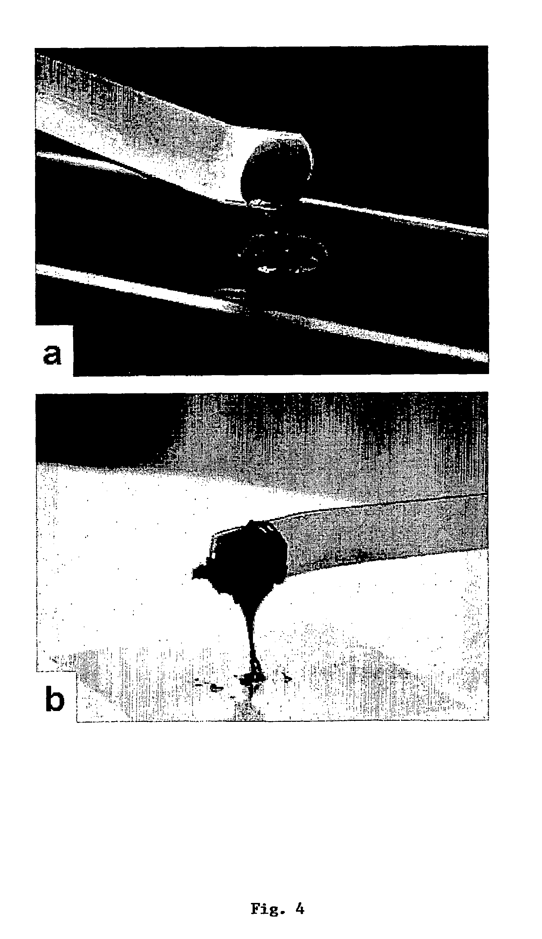

Figures 3 a and b show typical levels of glycosylated (a) and non-glycosylated (b) isoflavones found in a soy-based gel. Figure 4 shows a typical soybean-based hydrogel obtained with the methods described in Example 2.

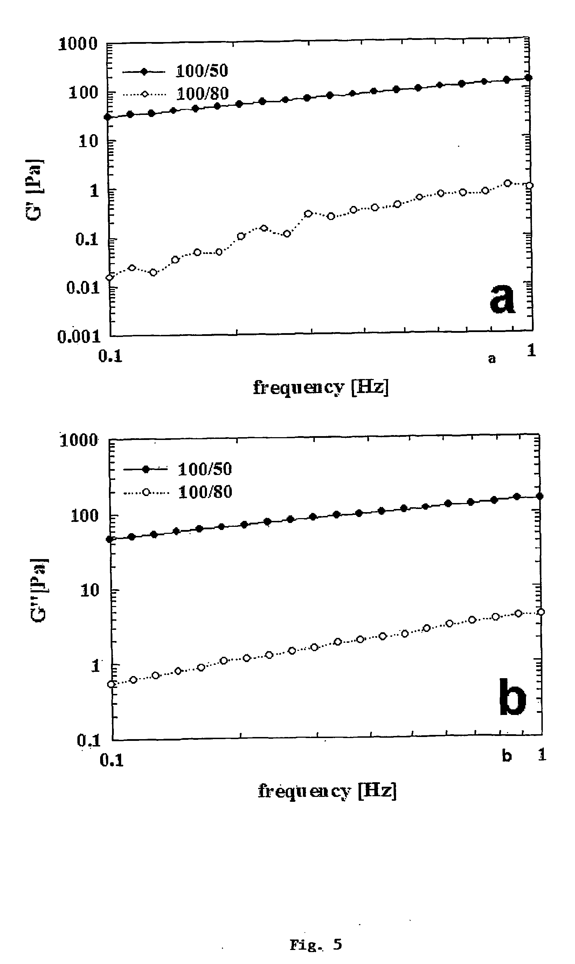

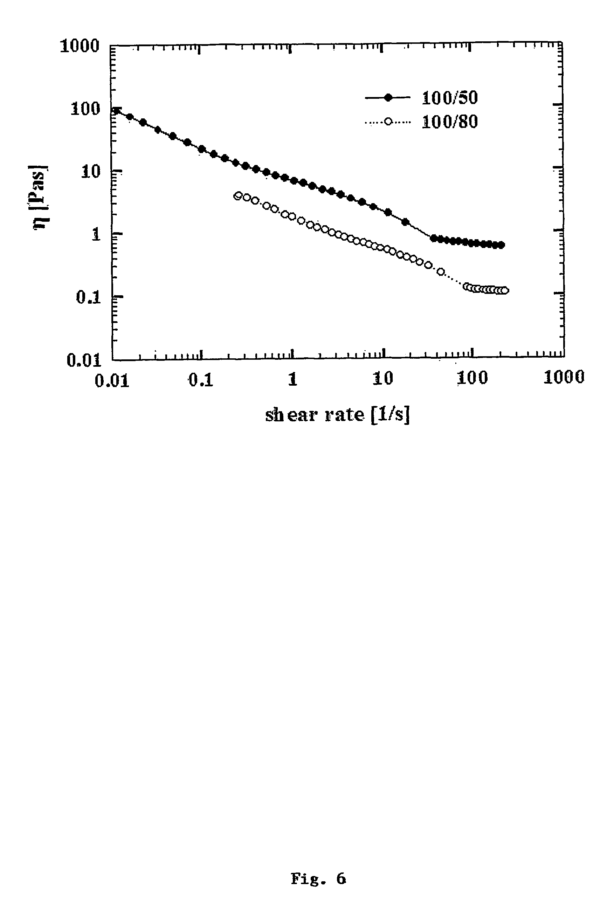

Figures 5 a and b and Figure 6 show the rheological properties of the soybean-based hydrogels at different concentrations of cross-linking agent (CaCl₂). When the cross-linking agent concentration was 0.1 M, it was possible to observe that for soybean hydrogels 0.1 M G" is always higher than G' indicating a viscous behaviour of the solution.

Viscosity measurements (Figure 6) suggest that viscosity changes are a function of the soybean biomaterial concentration; a more viscous material is obtained as soy extract content is increased. From a flow behaviour point of view (Figure 6), it is possible to observe that both concentrations show a pseudo-plastic behaviour. However, at high strain rates viscosity becomes less dependent on the strain rate and a Newtonian behaviour is detected. These rheological analysis data indicate that the hydrogels are suitable for injection.

### Example 3 - Method to obtain soy-based hydrogel biomaterials with controlled carbohydrate contents

### Methods

Soybean extracts were reconstituted in aqueous medium according to the two methods described in Example 2. After reconstitution and prior to cross-linking, the samples were dialyzed for different times against an excess of deionised water. A typical soy extract dialysis process consisted of, but was not limited to, incubation of samples in dialysis membranes with a molecular cut off of 8kDa in an excess of deionised water. The process was performed for up to 5 days, at room temperature, with stirring and a regular change of the dialysis medium. The dialysed fraction (protein-rich) and the external dialysis medium (carbohydrate fraction) were collected, freeze-dried and re-suspended in 1 ml of deionised water for analysis. Protein and carbohydrate amounts were assessed as described in Example 2 at different times to monitor the partial or complete removal of the carbohydrate fraction and the preservation of the protein fraction.

### Results

The Anthron assay of the samples retained within the dialysis membrane showed a progressive reduction of the carbohydrate fraction from 35% (initial value) to non-detectable values (protracted dialysis). The Bradford assay confirmed that no significant protein loss took place during the dialysis sample. The protein percentage in the final extract remained at around 53%. After freeze-drying, the powder obtained from the dialysis medium appeared to be of gluey consistency supporting the release of carbohydrate from the sample. This visual inspection was confirmed by the Anthron assay that showed a gradual increase of released carbohydrates over time. Visual inspection of the fraction retained within the dialysis membrane showed an increased density of the solution indicating that soybean biomaterials of different consistency could be obtained by removal of the carbohydrate fraction.

### Example 4 - Methods to obtain soy-based hydrogel biomaterials with controlled isoflavone contents

### Methods

Soybean hydrogels with controlled isoflavone contents were obtained by extractions performed following the method described in Example 2, while combining different solvent systems and temperatures. Examples of the solvent/temperature/time conditions are given in, but not limited to, Table I. The efficiency of the different extraction methods was tested gravitometrically, whereas the isoflavone content was assessed by HPLC. Given the solubility of the isoflavones in solvents such as dimethyl sulfoxide and methanol, biomaterials with different isoflavone compositions can also be prepared by introducing different percentages of these solvents in the extraction medium.

**Table I. Examples of typical extraction conditions to obtain soy-based hydrogels.**

| **Solvent system** | **Temperatures (°C)** | **Time (h)** | **Water (%)** |
|---|---|---|---|
| Methanol/Water | 30, 50 | 2,4 | 50,80 |
| Ethanol/Water | 30, 50 | 2,4 | 50,80 |
| Acetonitrile | 30, 50 | 2,4 | 50,80 |
| Acetone | 30,50 | 2,4 | 50,80 |

### Results

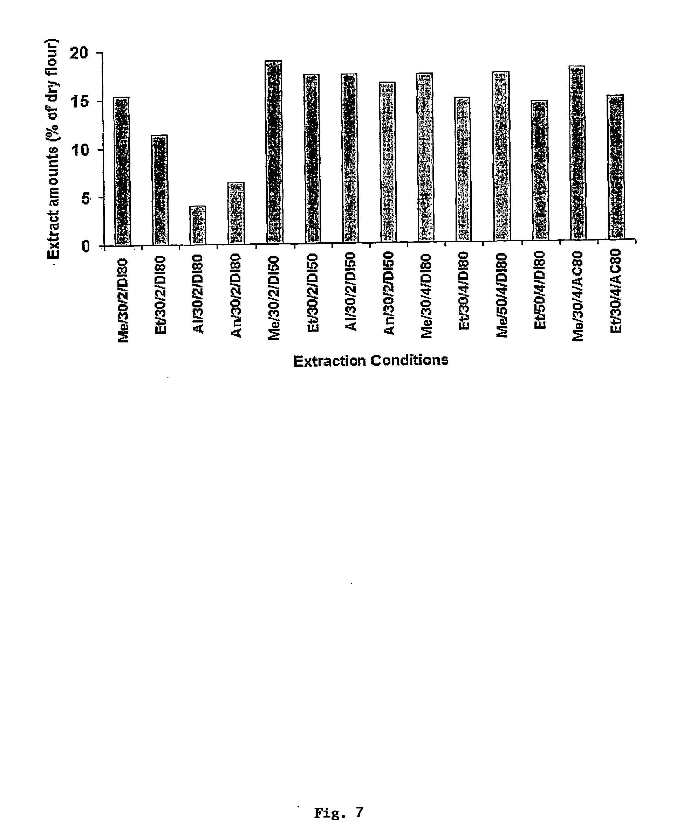

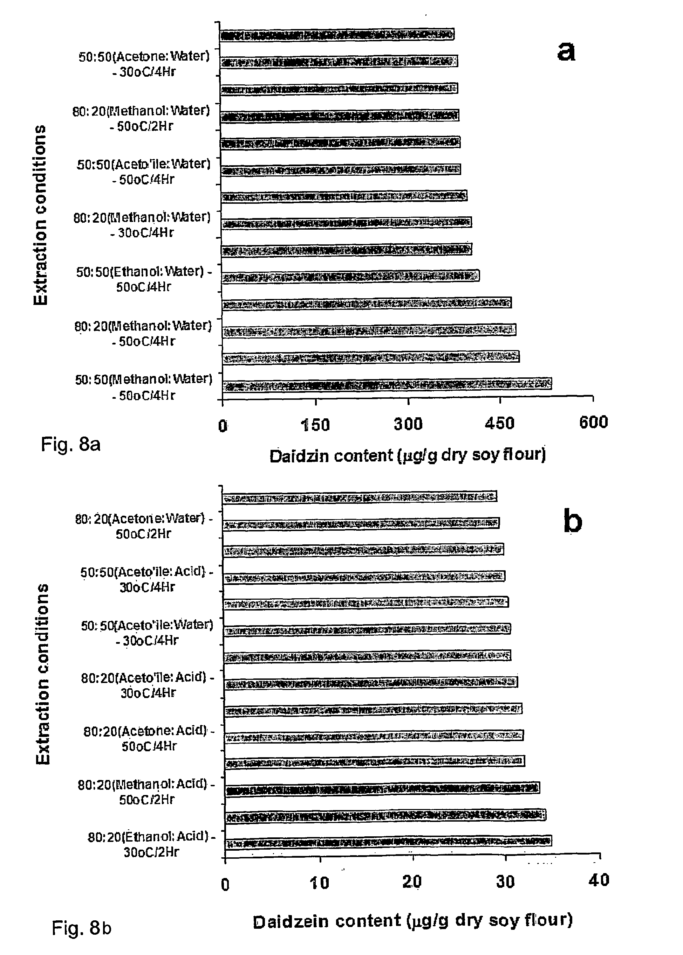

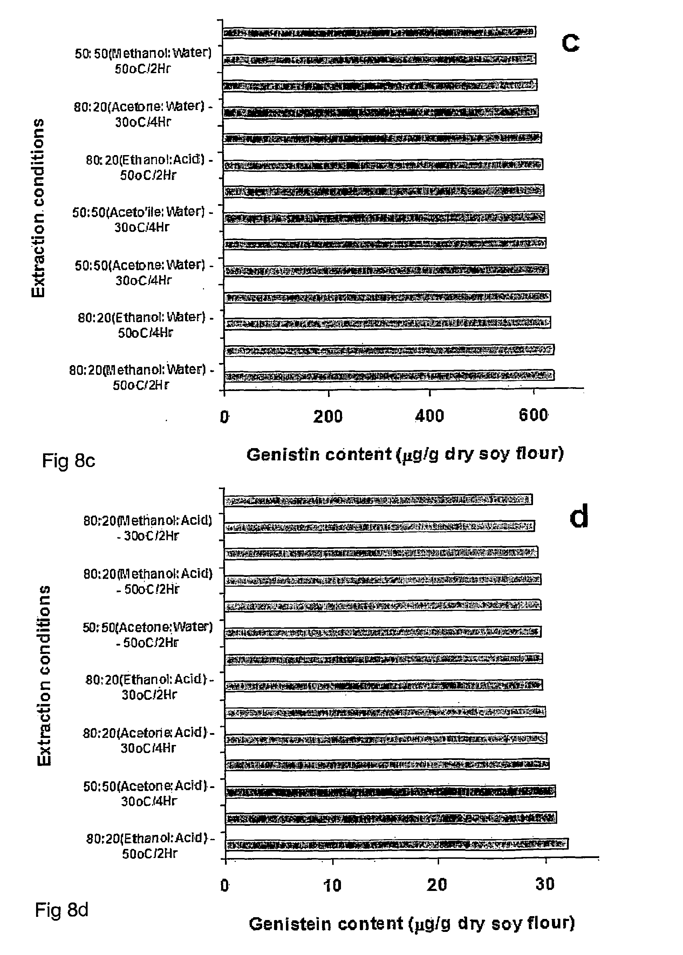

Figure 7 shows the amount of soy extract obtained from each type of extraction, showing that the level of extraction could be tuned in a relatively accurate manner. Figures 8 a-d show the HPLC isoflavone profiles of the different soybean-based hydrogels obtained from the combination of different solvent systems and temperatures. The graphs show that the isoflavone concentration can be controlled during the manufacturing process of the biomaterial, thus allowing to have gels with isoflavone levels tuned to the requirements of the final biomedical applications.

### Example 5 - Biomaterial blends, interpenetrated polymer networks and composites and functionalised scaffolds.

### Methods

De-fatted and thermoset soybean flour prepared as described in Example 1 as well as soybean-based hydrogels prepared as in Examples 2, 3 and 4 were blended with typical synthetic and natural polymers as well as with ceramic materials at different weight/weight ratios. The polymer blends and interpenetrated polymer networks include, but are not limited to, xanthan gum, polycaprolactones of different molecular weights, poly(ethylene glycol), poly(lactic/glycolic acid) of different copolymer ratios, agarose, alginates, chitosan, silk fibroin, fibrin glue and the like, as well as with linear and branching polymers (e.g. dendrimers).

Soybean-based biomaterials prepared as in the Examples 1, 2, 3 and 4 were mixed with different percentages of HA powder and beads as well as with calcium phosphate cements.

Soybean-based biomaterials prepared as in Examples 1, 2, 3 and 4 were mixed with several thickening substances such as, for example, xanthan gum.

Soybean-based biomaterials prepared as in Examples 1, 2, 3 and 4, alone or in combination with other biomaterials, were functionalised with peptides containing sequences recognised by different cell types (e.g. -RGD-, -FHRRIKA-, and others) and enzymes (e.g Factor XIIIa), synthesised with traditional peptide synthesis and grafted to the biomaterial by conventional biochemistry (e.g. Schiff's bases, thiol group and others) or by enzymatic activity.

Visual inspection and scanning electron microscopy (SEM) analysis were used to characterise the morphology of the different formulations.

### Results

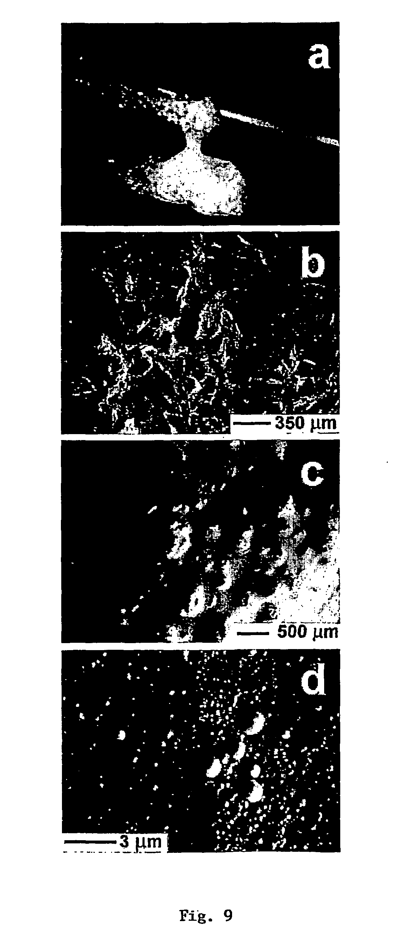

A series of blends of different material percentages could be obtained. Figures 9 a and b show the visual and SEM analysis of a typical polymer blend of the soybean-based biomaterials of Examples 2, 3 and 4 with another polymer (e.g. xanthan gum) and soy-based granules obtained by the methods used in Example 1 and described in WO 02/10261 (22). Figures 9 c and d show a typical example of the soybean-based biomaterials of Examples 2 to 4 mixed with ceramics materials such as hydroxyapatite in nanocrystalline (c) and bead (d) forms. Other ceramics, such as calcium phosphate cements and bioglasses, can also be used to this purpose.

### Example 6 - Controlled release of isoflavones from soybean-based biomaterials

### Methods

Soybean-based biomaterials prepared as in Examples 1, 2, 3 and 4 were incubated in phosphate buffered saline pH 7.4 (1 ml) for different times (6 to 120 hours) under static conditions. Supernatant aliquots (0.1 ml) were withdrawn and analysed by HPLC for their isoflavone content. The concentration of the different isoflavones released in the incubation medium was evaluated by the integration of the chromatogram peaks. Data were transformed from standard curves for each single isoflavone of interest and expressed as mg/ml ± standard deviation from n=3.

### Results

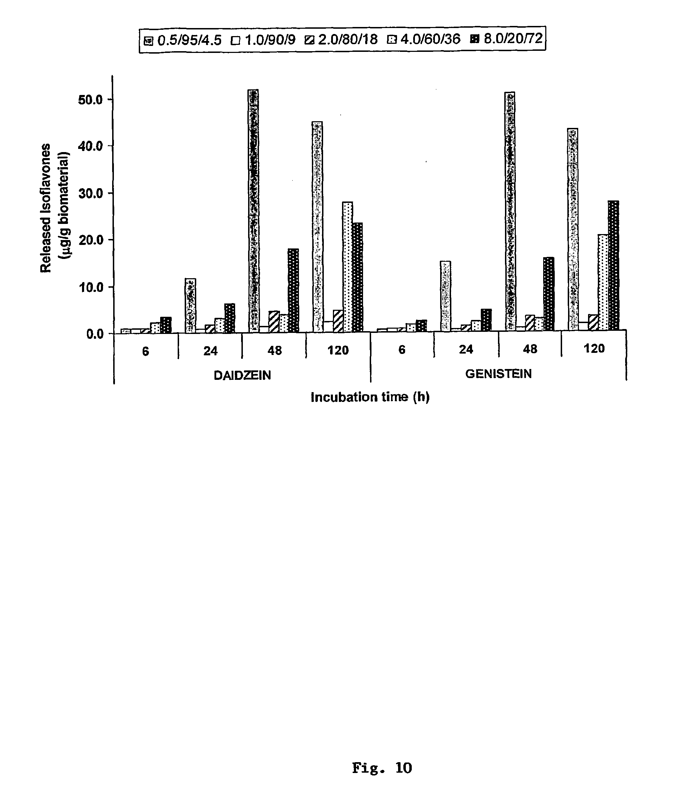

The data shown in Figure 10 indicate that the soybean-based biomaterials of Examples 1 and 2 are able to sustain the release of the main isoflavones at least over 120 hours of incubation.

### Example 7 - Soybean-based solids and hydrogels as biomaterials for manufacturing biomedical devices

### Methods

(a) Soy-based monolith biomaterials. Soybean-based monolith biomaterials prepared as described in Examples 1, 2, 3 and 4 were used alone or in combination with the soy-based biomaterials of WO 02/10261 and with other biomaterials to manufacture biomedical devices such as bone fillers (also as injectable gel), wound dressings (also as injectable gel), tissue sealants/bioglues (also as injectable gel), cartilage and bone scaffolds (also as injectable gel), nerve guides, cardiovascular stents and the like. Nonporous or porous blocks, granules, membranes and films were obtained by different degrees of packing of the biomaterial granules and gels prepared in Examples 1, 2, 3 and 4 in a mould. Porosity was also generated by mixing the biomaterials of Examples 1, 2, 3 and 4 with sugar and salt crystals as well as with polymer beads of different mesh. The additives were then rapidly dissolved by their solvent, leaving voids within the soy-based biomaterial. Moulding also allowed to shape the soy-based biomaterials to adapt them to specific biomedical applications. For example, soybean-based biomaterials prepared as in Examples 1, 2, 3 and 4 were thermoset around a metal wire which was removed after thermosetting to form a hollow fibre or tubing.
(b) Soybean-based biomaterials as coatings. Soybean-based biomaterials as prepared in WO 02/10261 and those prepared following the methods described in Examples 1, 2, 3 and 4 were used alone or in combination with other biomaterials to coat dental and orthopaedic implants, cardiovascular implants and nerve regeneration guides. Hydrogels, powders, films, sponge and the like can be deposited, mixed, grafted or folded around the medical device. For example, soft soybean hydrogels prepared as in Examples 2, 3 and 4 can be applied as a coating by a dip-coating procedure using the different formulations prepared as described in Example 4 capable of releasing isoflavones as described in Example 6. The coating can undergo a further stabilisation by a thermosetting process at temperatures above 60°C and/or by its incubation in cross-linking agents such as, but not limited to, divalent cations (e.g. calcium and magnesium solutions) or dialdehydes (e.g. glutaraldehyde and genipin). Alternatively, defatted soybean curd can be packed around the surface of a metal, polymeric or ceramic implant and then thermoset and/or chemically crosslinked to form coatings of different roughness, thickness and architectures. The success of the coating procedure was demonstrated by backscattering SEM at different magnifications.

The bioactivity of the materials obtained from methods (a) and (b) of this example was tested for their ability to stimulate the synthesis of collagen by fibroblasts and osteoblasts following a standard Sirius Red staining method. Osteoblast calcification potential was tested by using an Alizerin staining method, whereas the activation of osteoclast cells was assessed using a staining method to highlight the activity of the tartrate-resistant acid phosphatase enzyme in a culture system where osteoclasts were stimulated by colony stimulating factor as well as by co-culturing with osteoblasts. Soy-based biomaterials formulations, including but not limited to the soy-based gels of Example 2 mixed with the granules obtained following the method reported in Example 1 and of WO 02/10261, were also tested for their ability to participate in the blood clot formation by the biomaterials incubation in freshly collected blood and assessment of their integration in the forming clot by visual inspection and light microscopy.

### Results

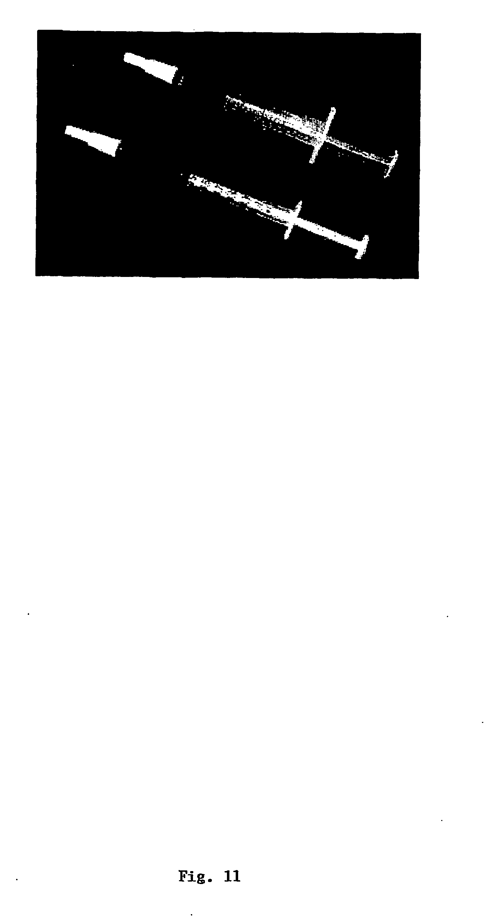

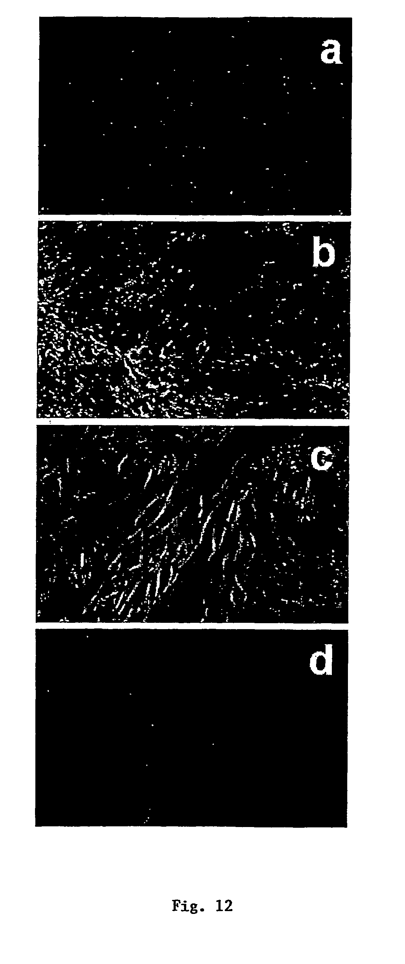

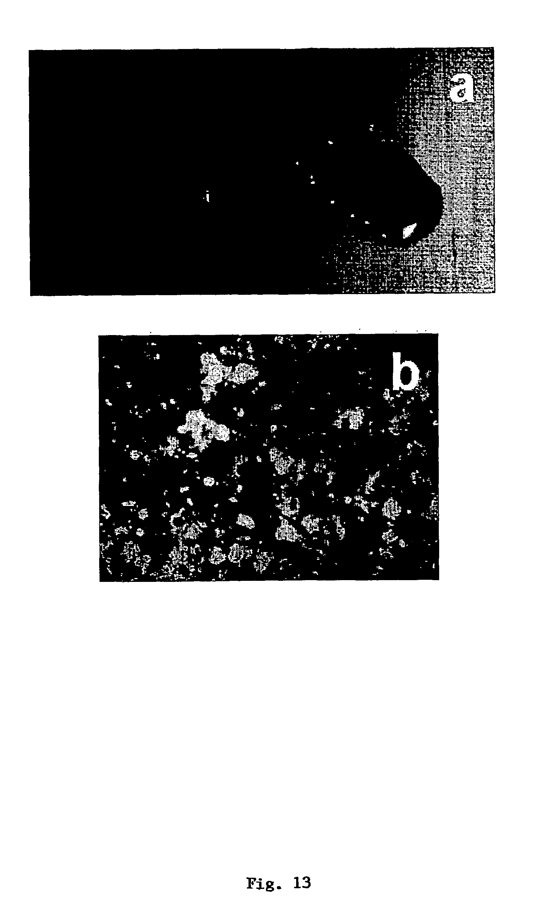

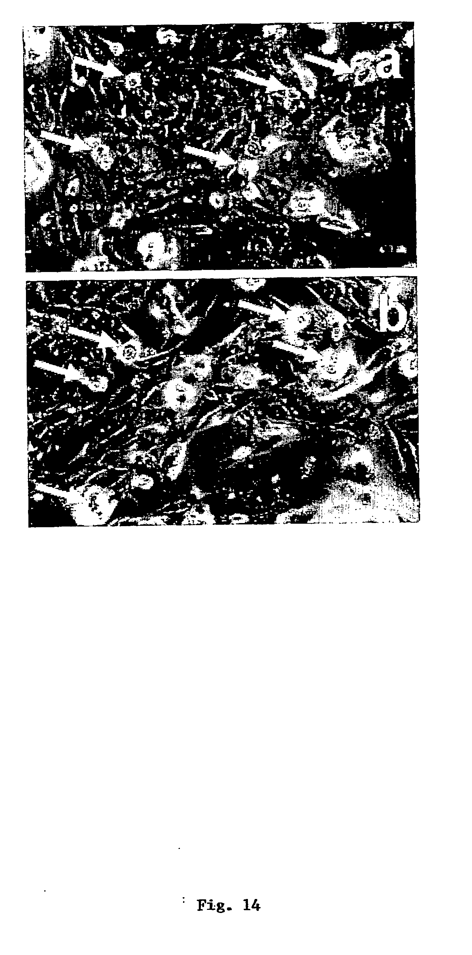

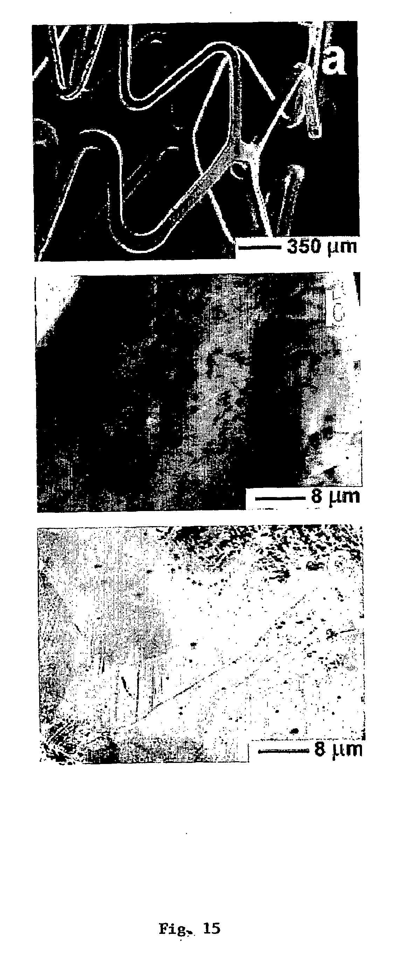

Wound dressings and bone fillers with different physico-chemical properties were obtained. Typical injectable gel formulations are shown in Figure 11. Both wound dressings and bone fillers showed a bioactivity which led to the induction of collagen synthesis by both fibroblasts and osteoblasts (Figures 12 a and b) and the calcification of osteoblast cultures (Figures 12 c and d). Tissue sealants (i.e. bioglues) were also, for example, obtained by mixing the soy-based biomaterial granules of WO 02/10261 or soy-based biomaterial granules of Example 1 with the soy-based hydrogels of Examples 2, 3 and 4 (Figures 9 a and b). Figures 13 a and b show that soy-based gel alone or in combination with other materials can be used as sealants (i.e. bioglues) as they favour the integration of the blood clot within their structure. Light microscopy showed that the clot invaded the gel and surrounded the granules dispersed in the gel during the preparation of this biomaterial formulation. Figures 14 a and b show the osteoclast tartrate-resistant acid phosphatase inhibitory effect of the soy-based biomaterials produced following the methods described in Examples 1, 2, 3 and 4. Arrows indicate the control osteoclasts positive to tartrate-resistant acid phosphatase (red-staining) after 8 days of co-culturing with osteoblasts (Figure 14 a). Figure 14 b shows cells with a pale red or yellow colour indicating an inhibition of the osteoclasts tartrate-resistant acid phosphatase in samples treated with the soybean biomaterials. Figures 15 a and c show a comparative SEM of a cardiovascular stent after its coating with soy-based hydrogels thermoset. The low magnification images show that the coating applied does not affect the stent strut design intimately adhering to its surface (Figure 15 a). Backscattering SEM also proves the successful apposition of the coating which appears as typical carbon-based, soft material at high magnification (Figure 15 b), while the uncoated surface clearly show its metal structure (Figure 15 c).

### Example 7. Soybean-based biomaterials as soothers

### Methods

Soybean-based biomaterials prepared as described in Examples 1, 2, 3 and 4 were used alone or in combination with the soy-based biomaterials of WO 02/10261 and with other biomaterials to manufacture soothers for irritated gums and skin. Hydrogels and pastes of different consistency were prepared following the methods described in the previous Examples and made suitable for spreading on tissues. Alternatively, the soybean-based biomaterials prepared as in Examples 2, 3 and 4 were made ready soluble by dissolution in an excess of aqueous solution at different ranges of concentrations (e.g.1 mg/ml and higher dilutions).

### Results

Hydrogels and pastes showed to be easily applicable on soft tissues such as skin and gums. In the case of the aqueous solutions, different concentrations of soybean-based biomaterials could be readily dissolved to obtain soothers with different bioactivity.

### References

1. Salgado AJ, Coutinho OP, Reis RL. Macromolecular Bioscience 4, 743-765 (2004)
2. Seliktar D, Zisch AH, Lutolf MP, Wrana JL, Hubbell JA. MMP-2 sensitive, VEGF-bearing bioactive hydrogels for promotion of vascular healing. J. Biomed. Mater. Res. Part A 68A, 704-712 (2004).
3. Wan ACA, Mao HQ, Wang S, Phua SH, Lee GP, Pan JS, Lu S, Wang J, Leong KW. Poly(phosphoester) ionomers as tissue-engineering scaffolds. J. Biomed. Mater. Res. Part B - Appl. Biomater. 70B, 91-102 (2004).
4. Luyten FP, Dell'Accio F, De Bari C. Skeletal tissue engineering: opportunities and challenges. Best Pract. Res. Clin. Reumatol. 15, 759-769 (2001).
5. Castner DG, Ratner BD. Biomedical surface science: foundation to frontiers. Surf. Sci. 500, 28-60 (2002).
6. Anderson JM, Shive MS. Biodegradation and biocompatibility of PLA and PLGA microspheres. Adv. Drug Deliv. Rev. 28, 5-24 (1997).
7. Gogolewski S, Pineda L, Busing CM. Bone regeneration in segmental defects with resorbable polymeric membranes: IV. Does the polymer chemical composition affect the healing process? Biomaterials 21, 2513-2520 (2000)
8. Zaffe D, Leghissa GC, Pradelli J, Botticelli AR. Histological study on sinus lift grafting by Fisiograft and Bio-Oss. J. Mater. Sci. : Mater. Med. 16, 789-793 (2005).
9. Noro T, Itoh K. Biomechanical behaviour of hydroxyapatite as bone substitute material in a loaded implant model. On the surface strain measurement and the maximum compression strength determination of material crash. Biomed. Mater. Eng. 9, 319-324 (1999).
10.Xu HHK, Quinn JB, Whisker-reinforced bioactive composites containing calcium phosphate cement fillers: effects of filler ratio and surface treatments on mechanical properties. J. Biomed. Mater. Res. 57, 165-174 (2001).
11. Yuasa T, Miyamoto Y, Kon M, Ishikawa K, Takechi M, Momota Y, Tatehara S. Takano H, Mimamiguchi S, Nagayama M. Proliferation and differentiation of cultured MC3T3-E1 osteoblasts on surface layer modified hydroxyapatite ceramic with acid and heat treatments. Dent. Mater. J. 24, 207-212 (2005).
12. Boccaccini AR, Blaker JJ, Maquet V, Jerome R, Blacher S, Roether JA. Biodegradable and bioactive polymer/Bioglass ® composite foams for tissue engineering scaffolds. Curr. Res. Adv. Mater. Processes Mater. Sci. Forum 494, 499-506 (2005).
13. Liang H, Shi B, Fairchild A, Cale T. Applications of plasma coatings in artificial joints: an overview. Vacuum 73, 317-326 (2004).
14. Brown R. Bioartificial implants: design and tissue engineering. In: Structural Biological Materials: design and structure - property relationships. Ed. Elices M, Pergamon Materials Series vol. 4. Pergamon 2000 Amsterdam Elsevier Science Ltd.
15. Daament WF, vanMoerkerk HThB, Hafmans T, Buttafoco L, Poot AA, Veerkamp JH, van Kuppevelt TH. Preparation and evaluation of molecularly-defined collagen-elastin-glycosaminoglycan scaffolds for tissue engineering. Biomaterials 24, 4001-4009 (2003).
16. Madihally SV, Matthew HWT. Porous chitosan scaffolds for tissue engineering. Biomaterials 20, 1133-1142 (1999).
17.Xu JY, Liu ZS, Erhan SZ, Carriere CJ. Cross-linkers control the viscoelastic properties of soybean oil-based biomaterials. J. Amer. Oil Chem. Soc. 81, 813-816 (2004).
18. Vaz CM, Fossen M, van Tuil RF, de Graaf LA, Reis RL, Cunha AM. Casein and soybean protein-based thermoplastics and composites as alternative biodegradable polymers for biomedical applications. J. Biomed. Mater. Res. Part A 65A, 60-70 (2003).
19. Christensen HR, Bruun SW, Frokiaer H. Antigenic specificity of serum antibodies in mice fed soy protein. Intern. Arch. Allergy Immunol. 132, 58-67 (2003).
20. Middleton E, Kandaswami C, Theoharides TC. The effects of plant flavonoids on mammalian cells: implications for inflammation, heart disease, and cancer. Pharmacol. Rev. 52, 673-751 (2000).
21.Barnes S, Blair HC. Genistein for use in inhibiting osteoclasts. US Patent No. 5,506,211.
22. Santin M, Nicolais L, Ambrosio L. Soybean-based biomaterials. PCT Patent Application No. PCT/GB01/03464 published as WO 02/10261.
23. Pandjaitan N, Hettiarachchy N, Ju ZY, Crandall P, Sneller C, Dombek D. Evaluation of genistin and genistein contents in soybean varieties and soy protein concentrate prepared with 3 basic methods. J. Food Sci. 65, 399-402 (2000).
24. Ramirez-Suarez JC, Xiong YL. Effect of transglutaminase-induced cross-linking on gelation of myofibrillar/soy protein mixtures. Meat Sci. 65, 899-907 (2003).
25. Fan JF, Saito M, Yanyan Z, Szesze T, Wang LJ, Tatsumi E, Li LT. Gel-forming ability and radical scavenging activity of soy protein hydrolysate treated with transglutaminase. J. Food Sci. 70, C87-C92 (2005).
26. Wiese KL, Sneider HE. Factors influencing soybean oil extraction rates. J. Amer. Oil Chem. Soc. 62, 618 (1985).
27. Lima ES, Abdalla DSP. High-performance liquid chromatography of fatty acids in biological samples. Anal. Chim. Acta 465, 81-91 (2002).

## Claims

1. A method of producing a soybean-based biomaterial directly from de-fatted soy flour which is suitable for use in a biomedical product, the method comprising:-
defatting soy flour; either prior to or at the same time as, performing a solvent extraction;
and thermosetting the defatted soy flour;
to produce a biomaterial comprising variable levels of soy proteins, carbohydrates and isoflavones.

2. A method as claimed in claim 1, wherein the solvent extraction is performed using a solvent system comprising methanol, ethanol, acetonitrile, acetone and water or a mixture thereof.

3. A method as claimed in claim 1 or claim 2, wherein the solvent extraction is performed for a period of between 2 and 4 hours and at a temperature of between 30°C and 50°C.

4. A method as claimed in any one of claims 1 to 3, which includes the step of introducing a cross-linking agent.

5. A method as claimed in any one of claims 1 to 4, which includes the step of dialysis for the purpose of controlling the level of carbohydrates in the biomaterial.

6. A method as claimed in any one of claims 1 to 5, which includes the step of functionalising the biomaterial with one or more bioactive peptides.

7. A method as claimed in any one of claims 1 to 6, wherein the soybean-based biomaterial is in the form of a hydrogel.

8. A method as claimed in claim 7, which includes the step of blending, interpenetrating, grafting or otherwise combining the soybean-based biomaterial with one or more natural or synthetic biocompatible biomaterials to form a composite biodegradable or biostable material.

9. Use in a biomedical product of a soybean-based biomaterial produced by the method of any one of claims 1 to 8.

10. Use as claimed in claim 9, where the soybean-based biomaterial is in the form of a hydrogel.

11. Use as claimed in claim 10, wherein the hydrogel is in injectable form.

12. Use as claimed in claim 9 or claim 10, wherein the biomedical product is a wound dressing; a scaffold for tissue engineering; a filler or implant for use in surgery; a temporary barrier for use in dental or surgical procedures or to prevent post-surgical tissue adherence; a carrier for the delivery of drugs, bioactive peptides or plasmids; an anti-inflammatory agent; a coating for wound dressings or for dental, medical, surgical or veterinary devices or implants; or a composition for soothing skin or gum irritation.

13. A biomedical product which is a scaffold for tissue engineering; a filler or implant for use in surgery; a temporary barrier for use in dental or surgical procedures or to prevent post-surgical tissue adherence; an anti-inflammatory agent; or a composition for soothing skin or gum irritation; and **characterised in that** it comprises a soybean-based biomaterial produced by a method as claimed in any one of claims 1 to 8.

14. A biomedical product which is a wound dressing; or a coating for wound dressings or for dental, medical, surgical or veterinary devices or implants; and **characterised in that** it comprises a soybean-based biomaterial produced by a method as claimed in any one of claims 1 to 8.

15. A biomedical product which is a carrier for the delivery of drugs, bioactive peptides or plasmids; and **characterised in that** it comprises a soybean-based biomaterial produced by a method as claimed in any one of claims 1 to 8.

## Patentansprüche

1. Verfahren zur Herstellung eines auf Sojabohnen basierenden Biomaterials direkt aus entfettetem Sojamehl, das zur Verwendung in einem biomedizinischen Produkt geeignet ist, das Verfahren umfassend:
das Entfetten des Sojamehls; entweder vor dem oder gleichzeitig mit dem Durchführen einer Lösungsmittelextraktion;
und das Thermofixieren des entfetteten Sojamehls;
um ein Biomaterial herzustellen, das variable Gehalte an Sojaproteinen, Kohlehydraten und Isoflavonen aufweist.

2. Verfahren gemäß Anspruch 1, wobei die Lösungsmittelextraktion mithilfe eines Lösungsmittelsystems durchgeführt wird, welches Methanol, Ethanol, Acetonitril, Aceton und Wasser oder eine Mischung von diesen umfasst.

3. Verfahren gemäß Anspruch 1 oder Anspruch 2, wobei die Lösungsmittelextraktion für eine Zeitdauer zwischen 2 und 4 Stunden und bei einer Temperatur zwischen 30°C und 50°C durchgeführt wird.

4. Verfahren gemäß einem der Ansprüche 1 bis 3, welches den Schritt des Einbringens eines Vernetzungsmittels einschließt.

5. Verfahren gemäß einem der Ansprüche 1 bis 4, welches den Schritt der Dialyse zum Zweck der Kontrolle des Gehalts an Kohlehydraten in dem Biomaterial einschließt.

6. Verfahren gemäß einem der Ansprüche 1 bis 5, welches den Schritt des Funktionalisierens des Biomaterials mit einem oder mehreren bioaktiven Peptiden einschließt.

7. Verfahren gemäß einem der Ansprüche 1 bis 6, wobei das auf Sojabohnen basierende Biomaterial in Form eines Hydrogels vorliegt.

8. Verfahren gemäß Anspruch 7, welches den Schritt des Vermischens, Durchdringens, Pfropfens oder anderweitigen Kombinierens des auf Sojabohnen basierenden Biomaterials mit einem oder mehreren natürlichen oder synthetischen biokompatiblen Biomaterialien einschließt, um ein zusammengesetztes biologisch abbaubares oder biologisch stabiles Material zu bilden.

9. Verwendung eines auf Sojabohnen basierenden Biomaterials, das durch das Verfahren gemäß einem der Ansprüche 1 bis 8 hergestellt wurde, in einem biomedizinischen Produkt.

10. Verwendung gemäß Anspruch 9, wobei das auf Sojabohnen basierende Biomaterial in Form eines Hydrogels vorliegt.

11. Verwendung gemäß Anspruch 10, wobei das Hydrogel in injektionsfähiger Form vorliegt.

12. Verwendung gemäß Anspruch 9 oder Anspruch 10, wobei es sich bei dem biomedizinischen Produkt um einen Wundverband; eine Matrize (Scaffold) zur biotechnologischen Gewebebearbeitung (Tissue Engineering); ein Füllmittel oder Implantat zur Verwendung in der Chirurgie; eine temporäre Barriere zur Verwendung in zahnmedizinischen oder chirurgischen Verfahren oder zur Verhinderung von post-chirurgischer Gewebeanhaftung; einen Träger zur Verabreichung von Arzneimitteln, bioaktiven Peptiden oder Plasmiden; einen entzündungshemmenden Wirkstoff; eine Beschichtung für Wundverbände oder für zahnmedizinische, medizinische, chirurgische oder veterinärmedizinische Geräte oder Implantate; oder eine Zusammensetzung zur Linderung von Haut- oder Zahnfleischreizungen handelt.

13. Biomedizinisches Produkt, bei dem es sich um eine Matrize (Scaffold) zur biotechnologischen Gewebebearbeitung (Tissue Engineering); ein Füllmittel oder Implantat zur Verwendung in der Chirurgie; eine temporäre Barriere zur Verwendung in zahnmedizinischen oder chirurgischen Verfahren oder zur Verhinderung von post-chirurgischer Gewebeanhaftung; einen entzündungshemmenden Wirkstoff; oder eine Zusammensetzung zur Linderung von Haut- oder Zahnfleischreizungen handelt und das **dadurch gekennzeichnet ist, dass** es ein auf Sojabohnen basierendes Biomaterial umfasst, das durch ein Verfahren gemäß einem der Ansprüche 1 bis 8 hergestellt wurde.

14. Biomedizinisches Produkt, bei dem es sich um einen Wundverband; oder eine Beschichtung für Wundverbände oder für zahnmedizinische, medizinische, chirurgische oder veterinärmedizinische Geräte oder Implantate handelt und das **dadurch gekennzeichnet ist, dass** es ein auf Sojabohnen basierendes Biomaterial umfasst, das durch ein Verfahren gemäß einem der Ansprüche 1 bis 8 hergestellt wurde.

15. Biomedizinisches Produkt, bei dem es sich um einen Träger zur Verabreichung von Arzneimitteln, bioaktiven Peptiden oder Plasmiden handelt und das **dadurch gekennzeichnet ist, dass** es ein auf Sojabohnen basierendes Biomaterial umfasst, das durch ein Verfahren gemäß einem der Ansprüche 1 bis 8 hergestellt wurde.

## Revendications

1. Un procédé pour produire un biomatériau à base de soja directement à partir de farine de soja dégraissée qui est adaptée pour une utilisation dans un produit biomédical, le procédé comprenant :
dégraisser la farine de soja ; soit avant, soit au moment d'effectuer une extraction par solvant ;
et thermodurcir la farine de soja dégraissée ;
pour produire un biomatériau comprenant divers niveaux de protéines de soja, d'hydrates de carbone et d'isoflavones.

2. Un procédé tel que revendiqué dans la revendication 1, dans lequel l'extraction par solvant est effectuée à l'aide d'un système de solvant comprenant le méthanol, l'éthanol, l'acétonitrile, l'acétone et l'eau ou un mélange de ceux-ci.

3. Un procédé tel que revendiqué dans la revendication 1 ou la revendication 2, dans lequel l'extraction par solvant est effectuée pendant une période allant de 2 à 4 heures et à une température allant de 30 °C à 50 °C.

4. Un procédé tel que revendiqué dans n'importe laquelle des revendications 1 à 3, qui inclut l'étape d'introduire un agent de réticulation.

5. Un procédé tel que revendiqué dans n'importe laquelle des revendications 1 à 4, qui inclut l'étape de dialyse dans le but de contrôler le niveau d'hydrates de carbone dans le biomatériau.

6. Un procédé tel que revendiqué dans n'importe laquelle des revendications 1 à 5, qui inclut l'étape de fonctionnaliser le biomatériau avec un ou plusieurs peptides bioactifs.

7. Un procédé tel que revendiqué dans n'importe laquelle des revendications 1 à 6, dans lequel le biomatériau à base de soja est sous la forme d'un hydrogel.

8. Un procédé tel que revendiqué dans la revendication 7, qui inclut l'étape de mélanger, d'interpénétrer, de greffer ou de combiner autrement le biomatériau à base de soja avec un ou plusieurs biomatériaux biocompatibles synthétiques ou naturels pour former un matériau biostable ou biodégradable composite.

9. Utilisation d'un produit biomédical d'un biomatériau à base de soja produit par le procédé de n'importe laquelle des revendications 1 à 8.

10. Utilisation telle que revendiquée dans la revendication 9, où le biomatériau à base de soja est sous la forme d'un hydrogel.

11. Utilisation telle que revendiquée dans la revendication 10, dans laquelle l'hydrogel est sous forme injectable.

12. Utilisation telle que revendiquée dans la revendication 9 ou la revendication 10, dans laquelle le produit biomédical est un pansement pour plaie ; un support pour l'ingénierie tissulaire ; un produit de remplissage ou un implant pour une utilisation en chirurgie ; une barrière temporaire pour une utilisation dans des interventions dentaires ou chirurgicales ou pour empêcher une adhérence tissulaire post-chirurgicale ; un véhicule pour l'administration de médicaments, de plasmides ou de peptides bioactifs ; un agent anti-inflammatoire ; un revêtement pour pansements pour plaie ou pour des implants ou des dispositifs dentaires, médicaux, chirurgicaux ou vétérinaires ; ou une composition pour soulager l'irritation des gencives ou de la peau.

13. Un produit biomédical qui est un support pour l'ingénierie tissulaire ; un produit de remplissage ou un implant pour une utilisation en chirurgie ; une barrière temporaire pour une utilisation dans des interventions dentaires ou chirurgicales ou pour empêcher une adhérence tissulaire post-chirurgicale ; un agent anti-inflammatoire ; ou une composition pour soulager l'irritation des gencives ou de la peau ; et **caractérisé en ce qu'**il comprend un biomatériau à base de soja produit par un procédé tel que revendiqué dans n'importe laquelle des revendications 1 à 8.

14. Un produit biomédical qui est un pansement pour plaie ; ou un revêtement pour pansements pour plaie ou pour des implants ou des dispositifs dentaires, médicaux, chirurgicaux ou vétérinaires ; et **caractérisé en ce qu'**il comprend un biomatériau à base de soja produit par un procédé tel que revendiqué dans n'importe laquelle des revendications 1 à 8.

15. Un produit biomédical qui est un véhicule pour l'administration de médicaments, de plasmides ou de peptides bioactifs ; et **caractérisé en ce qu'**il comprend un biomatériau à base de soja produit par un procédé tel que revendiqué dans n'importe laquelle des revendications 1 à 8.
